# FASCICULE DE BREVET EUROPEEN

(11) **EP 0 636 607 B1**
(45) Date de publication et mention de la délivrance du brevet: **22.01.1997**
(21) Numéro de dépôt: 94401707.8
(22) Date de dépôt: 26.07.1994
(51) Int. Cl.: C07C 319/28, C07C 323/58

(54) **Méthode de cristallisation de la méthionine**
Verfahren zum Kristallisieren von Methionin
Method of crystallising methionine

(30) Priorité: 28.07.1993 FR 9309273
(43) Date de publication de la demande: 01.02.1995
(73) Titulaire: RHONE-POULENC NUTRITION ANIMALE, 92160 Antony (FR)
(72) Inventeur: Giraud, Jean, F-30340 Salindres (FR); Leclaire, Daniel, F-03310 Durdat Larequille (FR)
(74) Mandataire: Le Pennec, Magali

(56) Documents cités:
- CHEMICAL ABSTRACTS, vol. 71, no. 9, 1 Septembre 1969, Columbus, Ohio, US; abstract no. 39393w, page 346; JP-A-43 024 890

## Description

La présente invention concerne un nouveau procédé de cristallisation de la DL-méthionine. La présente invention concerne plus particulièrement un procédé amélioré de cristallisation de la DL-méthionine par ajoût d'un additif de cristallisation à la solution contenant la méthionine préalablement à la cristallisation.

Il est connu selon le brevet japonais publié sous les numéro JP 68-024890 qu'il est possible d'améliorer la cristallisation de la méthionine par addition d'alcools, de phénols ou de cétones ayant 4 à 18 atomes de carbone. Il est également décrit dans le brevet japonais publié sous le numéro 71-019610 qu'il est possible de cristalliser la méthionine en ajoutant au milieu dans laquelle la méthionine est soluble un agent tensioactif anionique ou non-ionique. Il est enfin connu selon le brevet japonais publié sous le numéro 68-022285 qu'il est encore possible de cristalliser la méthionine à partir de solutions la contenant par ajoût de dérivés de la cellulose soluble dans l'eau.

La présente invention a permis d'améliorer la cristallisation de la méthionine à partir de solution de neutralisation du méthioninate de sodium en évitant la formation de produits amorphes donc poussiérant.

Elle consiste à ajouter à la solution aqueuse contenant la méthionine un sel d'aluminium d'un acide organique. Cet additif de cristallisation est choisi parmi les sels solubles dans l'eau de l'aluminium et d'un acide organique. L'acide organique est de préférence soluble dans l'eau et contient de préférence au maximum 8 atomes de carbone et encore plus préférentiellement au maximum 4 atomes de carbone. Il contient de préférence une ou plusieurs fonctions alcool et/ou acide.

On peut citer, non limitativement, à titre d'acide convenant dans le procédé de l'invention les acides suivants:
- l'acide lactique
- l'acide glycolique
- l'acide acétique
- l'acide malique
- l'acide tartrique
- l'acide citrique

On préfère tout particulièrement utiliser le lactate, le glycolate et l'acétate d'aluminium.

Les sels d'acides organiques et d'aluminium présentent l'avantage par rapport aux sels d'aluminium et d'acides minéraux de pouvoir être utilisés à un pH compris entre 4,5 et 7 et de préférence entre 4,5 et 5,5 sans voir apparaître une précipitation concommitante de l'hydroxyde d'aluminium.

La présente invention permet une cristallisation directe de la méthionine à partir d'une solution contenant de la méthionine et du sulfate de sodium dont le pH est d'environ 5 dans laquelle on ajoute une quantité suffisante d'un sel d'aluminium d'un acide organique.

Cette solution provient de l'acidification par l'acide sulfurique d'une solution contenant du méthioninate de sodium et du carbonate de sodium qui elle-même provient de l'hydrolyse de l'hydantoïne.

L'addition de sel organique d'aluminium à la solution de sulfate de sodium et de méthionine accompagnée d'un refroidissement jusqu'à environ 40°C permet d'obtenir des cristaux de méthionine sans apparition notable de trouble provoqué par l'hydroxyde d'aluminium et sans problème de filtration lors de l'isolement de la méthionine.

La quantité pondérale de sel organique d'aluminium exprimée en équivalent aluminium calculée par rapport à la méthionine, ajoutée au milieu de cristallisation est comprise entre 500 et 5000 ppm et plus préférentiellement entre 500 et 3000 ppm. Dans le cas du lactate d'aluminium, la quantité ajoutée lors de la cristallisation peut varier dans de larges limites sans influence sur la filtration subséquente alors que lors de l'utilisation du sulfate d'aluminium, l'augmentation de la quantité d'aluminium provoque des problèmes lors de la filtration. Ainsi, l'utilisation de lactate d'aluminium permet avantageusement des recyclages successifs des eaux mères de filtration ce qui n'est pas possible lors de l'utilisation du sulfate d'aluminium. La quantité de lactate utilisée sera adaptée par l'homme de l'art à l'économie du procédé, il est évident que des quantités trop élevées au-delà de 5000 ppm n'apportent aucun avantage technique supplémentaire et grèvent irrémédiablement l'économie du procédé.

Le sel organique d'aluminium peut être utilisé seul ou en associant deux sels différents.

La méthionine cristallisée obtenue est séparée par toute méthode appropriée telle que la filtration, la centrifugation. Les cristaux obtenus contiennent moins d'eau que ceux obtenus en l'absence d'additif, ils ont la forme de grains et présentent une densité d'environ 0,50 bien supérieure à celle de la méthionine obtenue sans additif.

La présente invention sera plus complètement décrite à l'aide des exemples suivants.

### EXEMPLE 1

Dans un récipient d'un litre contenant 650 g d'une solution aqueuse contenant 60 g de DL-méthionine et 98,1 g de sulfate de sodium maintenue à 100°C dont le pH est de 5 on ajoute 0,327 g de lactate d'aluminium en solution à 20 % dans l'eau.

On refroidit lentement le milieu de cristallisation en agitant à 300 tours/minutes jusqu'à 40°C. On récupère 39,1 g de cristaux de méthionine dont les caractéristiques sont indiquées dans le tableau 1.

### EXEMPLE 2 ET 3

On reproduit l'exemple 1 en remplaçant la lactate d'aluminium par 0,132 g d'acétate d'aluminim ou 0,28 g de glycolate d'aluminium en solution à 15 % dans l'eau. Les résultats sont indiqués dans le tableau 1.

### EXEMPLES 4 A 7

Sur une solution neutralisée de saponification de l'hydantoïne contenant 9,2 % en poids de méthionine et 15 % en poids de sulfate de sodium maintenue à 100°C sous pression atmosphérique ayant un pH compris entre 5 et 5,5 on ajoute 1000 à 3000 ppm par rapport à la méthionine d'aluminium sous forme de lactate ou de sulfate.

On observe la présence ou l'absence de l'apparition d'un précipité.

**TABLEAU 1**

| | | | Caractéristiques de la méthionine | | |
|---|---|---|---|---|---|
| Essais | Additifs | Taux ppm d'Al/MTN | Perte au séchage % | Densité | Aspect des cristaux |
| | sans (témoin) | 0 | 35,2 | 0,27 | lamelles amorphes |
| 1 | Lactate | 500 | 25 | 0,52 | petits grains |
| 2 | Acétate | 500 | 22,2 | 0,55 | petits grains |
| 3 | Glycolate | 500 | 27,9 | 0,51 | petits grains |

**TABLEAU 2**

| Essais | PH | Taux Al ppm/MTN | Additif | Solution | Filtration |
|---|---|---|---|---|---|
| C4 | 5 | 3000 | Sulfate | Trouble | lente + mousse |
| 4 | 5 | 3000 | Lactate | Limpide | normale |
| C5 | 5 | 2000 | Sulfate | Trouble | lente (30 mn) |
| 5 | 5 | 2000 | Lactate | Limpide | normale |
| C6 | 5,5 | 3000 | Sulfate | Trouble | très difficile (2 h 30) |
| 6 | 5,5 | 3000 | Lactate | Limpide | normale |
| C7 | 5,5 | 1000 | Sulfate | Trouble | normale |
| 7 | 5,5 | 1000 | Lactate | Limpide | normale |

## Revendications

1. Procédé de cristallisation de la méthionine caractérisé en ce qu'on réalise la cristallisation en présence d'un sel d'aluminium d'un acide organique.

2. Procédé selon la revendication 1 caractérisé en ce que l'acide organique est soluble dans l'eau.

3. Procédé selon les revendications 1 et 2 caractérisé en ce que l'acide organique contient au maximum 8 atomes de carbone et de préférence au maximum 4 atomes de carbone.

4. Procédé selon l'une quelconque des revendications 1 à 3 caractérisé en ce que que l'acide organique est un acide alcool.

5. Procédé selon l'une quelconque des revendications 1 à 4 caractérisé en ce que l'acide organique est un polyacide carboxylique.

6. Procédé selon l'une quelconque des revendications 1 à 5 caractérisé en ce que l'acide organique est choisi parmi l'acide lactique, l'acide glycolique et l'acide acétique.

7. Procédé selon l'une quelconque des revendications 1 à 6 caractérisé en ce que la quantité pondérale de sel d'aluminium de l'acide organique exprimé en équivalent aluminium calculée par rapport à la méthionine est comprise entre 500 et 5000 ppm et de préférence entre 500 et 3000 ppm.

8. Procédé selon l'une quelconque des revendications précédentes caractérisé en ce que le pH de la solution de cristallisation est compris entre 4,5 et 7 et de préférence entre 4,5 et 5,5.

## Patentansprüche

1. Verfahren zur Kristallisation von Methionin, dadurch gekennzeichnet, daß man die Kristallisation in Anwesenheit eines Aluminiumsalzes von einer organischen Säure realisiert.

2. Verfahren nach Anspruch 1, dadurch gekennzeichnet, daß die organische Säure in Wasser löslich ist.

3. Verfahren nach Anspruch 1 und 2, dadurch gekennzeichnet, daß die organische Säure höchstens 8 Kohlenstoffatome und vorzugsweise höchstens 4 Kohlenstoffatome enthält.

4. Verfahren nach irgendeinem der Ansprüche 1 bis 3, dadurch gekennzeichnet, daß die organische Säure eine Alkoholsäure ist.

5. Verfahren nach irgendeinem der Ansprüche 1 bis 4, dadurch gekennzeichnet, daß die organische Säure eine Polycarbonsäure ist.

6. Verfahren nach irgendeinem der Ansprüche 1 bis 5, dadurch gekennzeichnet, daß die organische Säure unter Milchsäure, Glycolsäure und Essigsäure ausgewählt wird.

7. Verfahren nach irgendeinem der Ansprüche 1 bis 6, dadurch gekennzeichnet, daß die Gewichtsmenge des Aluminiumsalzes der organischen Säure, ausgedrückt in Aluminiumäquivalent und berechnet im Verhältnis zum Methionin, zwischen 500 und 5000 ppm, vorzugsweise zwischen 500 und 3000 ppm beträgt.

8. Verfahren nach irgendeinem der vorstehenden Ansprüche, dadurch gekennzeichnet, daß der pH-Wert der Kristallisationslösung zwischen 4,5 und 7 und vorzugsweise zwischen 4,5 und 5,5 liegt.

## Claims

1. Process for the crystallization of methionine, characterized in that the crystallization is carried out in the presence of an aluminium salt of an organic acid.

2. Process according to claim 1, characterized in that the organic acid is soluble in water.

3. Process according to claims 1 and 2, characterized in that the organic acid contains at most 8 carbon atoms and preferably at most 4 carbon atoms.

4. Process according to any one of claims 1 to 3, characterized in that the organic acid is an acid alcohol.

5. Process according to any one of claims 1 to 4, characterized in that the organic acid is a polycarboxylic acid.

6. Process according to any one of claims 1 to 5, characterized in that the organic acid is chosen from lactic acid, glycolic acid and acetic acid.

7. Process according to any one of claims 1 to 6, characterized in that the amount by weight of aluminium salt of the organic acid, expressed as aluminium equivalent calculated with respect to the methionine, is between 500 and 5000 ppm and preferably between 500 and 3000 ppm.

8. Process according to any one of the preceding claims, characterized in that the pH of the crystallization solution is between 4.5 and 7 and preferably between 4.5 and 5.5.
